# FASCICULE DE BREVET EUROPEEN

(11) **EP 2 328 862 B1**
(45) Date de publication et mention de la délivrance du brevet: **21.10.2015**
(21) Numéro de dépôt: 09783247.1
(22) Date de dépôt: 21.09.2009
(51) Int. Cl.: C07C 233/05, C11D 7/50, A01N 25/02, C09D 7/12, C09D 11/00, C08K 5/20, C10M 133/16, C11D 3/43, A01P 3/00, A01P 7/04, A01P 11/00, A01P 13/00, A01P 21/00

(54) **PRODUIT COMPRENANT DES DIAMIDES, PROCEDE DE PREPARATION ET UTILISATIONS**
DIAMIDE ENTHALTENDES PRODUKT, VERFAHREN ZU DESSEN HERSTELLUNG UND ANWENDUNGEN DAVON
PRODUCT CONTAINING DIAMIDES, METHOD FOR MAKING SAME AND USES THEREOF

(30) Priorité: 22.09.2008 FR 0805188
(43) Date de publication de la demande: 08.06.2011
(73) Titulaire: Rhodia Opérations, 93306 Aubervilliers (FR)
(72) Inventeur: GUGLIERI, Massimo, 75005 Paris (FR)
(74) Mandataire: Jacobson, Claude
(86) Numéro de dépôt international: PCT/EP2009/062208
(87) Numéro de publication internationale: WO 2010/031867

(56) Documents cités:
- WO-A-01/89592
- WO-A-2008/074837

## Description

La présente invention a pour objet de nouveaux produits comprenant des composés diamides, des utilisations des produits et au moins un procédé de préparation. Ces produits peuvent notamment être utilisés comme solvants, par exemple dans des formulations phytosanitaires.

L'industrie utilise de nombreux produits chimiques à titre de solvants, par exemple pour préparer des produits chimiques et des matériaux, pour formuler des composés chimiques, ou pour traiter des surfaces. Par exemple des solvants sont utilisés pour la formulation d'actifs phytosanitaires notamment sous forme de concentrés émulsionnables (Emulsifiable Concentrate "EC") destinés à être dilués dans de l'eau par l'exploitant agricole, avant application sur un champ.

L'industrie est à la recherche de nouveaux produits permettant de varier ou d'optimiser les produits et procédés dans lesquels des solvants, notamment des solvants polaires, sont à utiliser. L'industrie a notamment besoin de composés de coût modeste, présentant des propriétés d'usage intéressantes. L'industrie a également besoin de composés présentant un profile toxicologique et/ou écologique perçu comme favorable, notamment une faible volatilité (faible VOC), une bonne biodégradabilité, une faible toxicité et/ou une faible dangerosité.

On connaît l'utilisation comme solvants des dialkylamides. Il s'agit de produit de formule R-CONMe₂ ou R est un groupe hydrocarbonné comme un alkyle, typiquement en C₆-C₃₀. De tels produits sont notamment commercialisés sous la dénomination Genagen® par la société Clariant. Ces solvants trouvent des applications notamment dans le domaine phytosanitaire.

On connaît également comme solvants les diesters d'acides dicarboxyliques, notamment les diesters obtenus par estérification d'un mélange d'acide adipique, d'acide glutarique et d'acide succinique. De tels produits sont notamment commercialisés sous les dénominations Rhodiasolv® RPDE et Rhodiasovl® DIB par la société Rhodia.

Les documents US 3288794 et US 3417114 décrivent des composés diamides d'acides dicarboxyliques linéaires de formule HOOC-(CH₂)_{z}-COOH comme l'acide adipique (z=4), l'acide glutarique (z=3), l'acide succinique (z=2). Ces composés sont solides, les points de fusion sont de l'ordre de 80°C. Ils ne peuvent donc pas être utilisés comme solvants à des températures plus modestes, notamment à température ambiante. De telles températures de fusion rendent par ailleurs difficiles leur manipulation et leur utilisation et impliqueraient des procédures à chaud.

Le document EP 186950 décrit des bis(amides) d'acides dicarboxyliques, obtenus à partir d'un monoamide, de CO et d'une amine. Ce document décrit en particulier la préparation de l'adipamide de formule H₂NOC-(CH₂)_{z}-CONH₂ où z=4. Le document enseigne l'utilisation des composés comme monomères ou intermédiaires destinés à la préparation de polymères.

Le document US 4588833 décrit la préparation d'amides d'acide succinique substitué. Ce document décrit en particulier la préparation de composés de type XOC-CH₂-CHR⁶-CONEt₂ où R⁶ est un méthyle ou un éthyle. Les produits sont préparés par mise en présence de CO, d'un alcool ou d'une amine HX, et d'acide crotonique dialkylamide ou d'acide pent-3-enoique diethylamide. Le document enseigne l'utilisation des composés comme anti-oxidants, comme stabilisants pour plastiques, ou comme intermédiaires de synthèse organique.

Il demeure un besoin, comme expliqué plus haut, pour d'autres produits, liquides, pouvant être utiles notamment comme solvants et pouvant faciliter la manipulation et les utilisations, sans forcement nécessiter de procédures à chaud.

On décrit ici des produits comprenant au moins deux composés diamides choisis parmi les composés diamides de formules (la), (Ib) et (Ic) suivantes :

R²R³NOC-A^{a}-CONR⁴R⁵ (Ia)

R²R³NOC-A^{b}-CONR⁴R⁵ (Ib)

R²R³NOC-A^{c}-CONR⁴R⁵ (Ic)

Où:
- R², R³, R⁴ et R⁵, identiques ou différents, sont des groupes choisis parmi les groupes hydrocarbonés comprenant un nombre moyen d'atomes de carbone allant de 1 à 36, saturés ou insaturés, linéaires ou ramifiés, éventuellement cycliques, éventuellement aromatiques, éventuellement substitués, R² et R³ d'une part et R⁴ et R⁵ d'autre part pouvant éventuellement former ensemble un cycle, éventuellement substitué et/ou comprenant éventuellement un hétéroatome, et
- A^{a}, A^{b}, et A^{c} sont des groupes alkyles divalents linéaires, comprenant chacun un nombre différent d'atomes de carbone.

On a trouvé de manière surprenante que des mélanges ou associations pouvaient être liquides alors que les composants sont individuellement solides.

La présente invention concerne un produit comprenant au moins deux composés diamides choisis parmi les composés diamides de formules (la), (Ib) et (Ic) susmentionnées dans lesquelles :
- R², R³, R⁴ et R⁵, identiques ou différents, sont choisis parmi les groupes méthyle, éthyle, propyle (n-propyl), isopropyle, n-butyle, isobutyle, n-pentyle, amyle, isoamyle, hexyle, cyclohexyle, hydroxyéthyle, morpholine, pipérazine ou pipéridine ;
- A^{a} est le groupe de formule suivante -CH₂-CH₂- (éthylène)
- A^{b} est le groupe de formule suivante -CH₂-CH₂-CH₂- (n-propylène), et
- A^{c} est le groupe de formule suivante -CH₂-CH₂-CH₂-CH₂- (n-butylène).

L'invention a également pour objet un procédé de préparation du produit de l'invention.

L'invention a également pour objet l'utilisation du produit de l'invention dans des formulations. L'invention a également pour objet un procédé de préparation des formulations par ajout du produit de l'invention. L'invention a également pour objet des formulations comprenant le produit de l'invention. Les formulations peuvent être notamment des formulations phytosanitaires.

L'invention a également pour objet l'utilisation du produit de l'invention comme solvant, plastifiant, ou agent de coalescence.

### Définitions

Dans la présente demande le terme solvant est entendu dans un sens large, couvrant notamment les fonctions de co-solvant, d'inhibiteur de cristallisation, de décapant. Le terme solvant peut notamment désigner un produit liquide à la température d'utilisation, de préférence de point de fusion inférieur ou égal à 20°C, de préférence à 5°C, de préférence à 0°C, pouvant contribuer à rendre liquide une matière solide, ou à empêcher ou retarder la solidification ou la cristallisation de matière dans un milieu liquide.

Dans la présente demande un composé répondant à une formule chimique désigne tout composé répondant à ladite. On mentionne que le terme "composé" couvre également des mélanges de plusieurs molécules répondant à une même formule. Il peut donc s'agir d'une molécule de ladite formule, ou d'un mélange de plusieurs molécules répondant à ladite formule. Par exemple un composé de formule (la) peut être une molécule de formule (la) avec des groupes R², R³, R⁴ et R⁵ définis, ou un mélange de plusieurs molécules répondant à la formule (la) lesdites molécules présentant entre elles des différences en ce qui concerne les groupes R², R³, R⁴ et R⁵

Dans la présente demande, par "composition de matière", on entend une composition, plus ou moins complexe, comprenant plusieurs composés chimiques. Il peut s'agir typiquement d'un produit de réaction non purifié ou modestement purifié. Le composé de l'invention pourra notamment être isolé et/ou commercialisé et/ou utilisé sous forme d'une composition de matière le comprenant. Le produit de l'invention est une composition de matière. Dans la composition de matière l'ensemble des composés de formules (la), (Ib) et (Ic) peuvent représenter au moins 10% en poids. De préférence, l'ensemble de ces composés constituent le produit principal de la composition de matière. Par produit principal, on entend dans la présente demande, le produit dont la teneur est la plus élevée, même si sa teneur est inférieure à 50% en poids (par exemple dans un mélange de 40% de A, de 30% de B, et de 30% de C, le produit A est le produit principal). Encore plus préférablement dans la composition de matière l'ensemble des composés de formules (Ia), (Ib) et (Ic) représente au moins 50% en poids de la composition de matière, par exemple de 70 à 95% en poids, et même de 75 à 90% en poids.

### Composés diamides

Le produit peut comprendre au moins deux des composés de formules (Ia), (Ib) et (Ic). Il comprend de préférence les trois composés.

Dans les formules (Ia), (Ib) et (Ic), A^{a}, A^{b}, et A^{c} sont des groupes alkyles divalents linéaires, comprenant chacun un nombre différent d'atomes de carbone. Les groupes A^{a}, A^{b}, et A^{c} correspondent à des diacides de formule HOOC-A^{a}-COOH, HOOC-A^{b}-COOH, et HOOC-A^{c}-COOH. Les nombres d'atomes de carbone peuvent être choisis notamment parmi 2 (acide correspondant = acide succinique), 3 (acide correspondant = acide glutarique), 4 (acide correspondant = acide adipique), 5, 6, 7, 8, 9, 10.

Selon l'invention :
- A^{a} est le groupe de formule suivante -CH₂-CH₂- (éthylène, acide correspondant = acide succinique)
- A^{b} est le groupe de formule suivante -CH₂-CH₂-CH₂- (n-propylène, acide correspondant = acide glutarique)
- A^{c} est le groupe de formule suivante -CH₂-CH₂-CH₂-CH₂- (n-butylène, acide correspondant = acide adipique).

Le produit comprend de préférence les trois composés diamides avec ces groupes particuliers A^{a} = éthylène, A^{b} = n-propylène, et A^{c} = n-butylène.

Selon un mode de réalisation intéressant les proportions des composés de formules (la), (Ib) et (Ic), par rapport au nombre total de moles de diamides de formules (la), (Ib), et (Ic), sont les suivantes:
- de 1 à 20%, de préférence de 5 à 15%, en moles, de diamide de formule (Ic)
- de 45 à 75%, de préférence de 55 à 65%, en moles, de diamide de formule (Ib), et
- de 15 à 45%, de préférence de 20 à 33%, en moles, de diamide de formule (Ia).

Dans le cas où A^{a} = éthylène, A^{b} = n-propylène, et A^{c} = n-butylène, le mélange des composés dans ces proportions peut correspondre (en version amide) à un mélange d'acides Adipique, Glutarique et Succinique ("AGS") disponible en grandes quantités comme sous produit de la préparation d'acide adipique, un monomère utilisé dans la fabrication de polyamide ou de polyurethane. Le mélange de diacides ou de diesters obtenus à partir du mélange de diacides, peut constituer une matière première particulièrement intéressante d'un point de vue économique, industriel, et environnemental car on utilise un sous produit et on optimise l'utilisation de ressources.

Dans les formules susmentionnées, les groupes R², R³, R⁴ et R⁵, identiques ou différents, sont des groupes choisis parmi les groupes hydrocarbonés comprenant un nombre moyen d'atomes de carbone allant de 1 à 36, saturés ou insaturés, linéaires ou ramifiés, éventuellement cycliques, éventuellement aromatiques, éventuellement substitués, R² et R³ d'une part et R⁴ et R⁵ d'autre part pouvant éventuellement former ensemble un cycle, éventuellement substitué et/ou comprenant éventuellement un hétéroatome. Le cycle comprend typiquement l'atome d'azote auquel R² et R³ ou R⁴ et R⁵ sont liés. Le cycle peut comprendre un hétéroatome supplémentaire. Le substituant peut être un groupe hydroxy.

Selon un mode de réalisation particulier les couples R² et R³ d'une part et R⁴ et R⁵ d'autre part sont des couples identiques. Selon un mode particulier R² et R³ sont identiques. Selon un mode particulier R⁴ et R⁵ sont identiques. Selon un mode particulier R², R³, R⁴ et R⁵ sont identiques

Selon l'invention, les groupes R², R³, R⁴ et R⁵, identiques ou différents, sont choisis parmi les groupes méthyle, éthyle, propyle (n-propyl), isopropyle, n-butyle, isobutyle, n-pentyle, amyle, isoamyle, hexyle, cyclohexyle, hydroxyéthyle. Les groupes R² et R³ peuvent également être tels qu'ils forment ensemble avec l'atome d'azote un groupe morpholine, piperazine ou piperidine. Selon des modes particuliers de réalisation, R²=R³=R⁴=R⁵=méthyle, ou R²=R³=R⁴=R⁵=éthyle, ou R²=R³=R⁴=R⁵=hydroxyéthyle.

Le produit de l'invention peut notamment être tel que:
- le composé de formule (la) est le composé Me₂NOC-CH₂-CH₂-CONMe₂
- le composé de formule (Ib) est le composé Me₂NOC-CH₂-CH₂-CH₂-CONMe₂, et
- le composé de formule (Ic) est le composé Me₂NOC-CH₂-CH₂- CH₂-CH₂-CONMe₂,

Le produit de l'invention peut notamment être tel que:
- le composé de formule (la) est le composé Et₂NOC-CH₂-CH₂-CONEt₂
- le composé de formule (Ib) est le composé Et₂NOC-CH₂-CH₂-CH₂-CONEt₂, et
- le composé de formule (Ic) est le composé Et₂NOC-CH₂-CH₂- CH₂-CH₂-CONEt₂.

Selon un mode de réalisation le produit est différent du produit constitué de
- 33% en poids ou en moles du composé
   (HOCH₂CH₂)HNOC-CH₂-CH₂-CONH(CH₂CH₂OH), et
- 67% en poids ou en moles du composé
   (HOCH₂CH₂)HNOC-CH₂- CH₂-CH₂-CONH(CH₂CH₂OH).

Selon un mode de réalisation le produit est différent du produit constitué de
- 33% en poids ou en moles du composé
   (HOCH₂CH₂)(CH₃)NOC-CH₂-CH₂-CON(CH₃)(CH₂CH₂OH), et
- 67% en poids ou en moles du composé
   (HOCH₂CH₂)(CH₃)NOC-CH₂- CH₂-CH₂- CON(CH₃)(CH₂CH₂OH).

Selon un mode de réalisation le produit est différent du produit constitué de
- 33% en poids ou en moles du composé
   (HOCH₂CH₂)₂NOC-CH₂-CH₂-CON(CH₂CH₂OH)₂, et
- 67% en poids ou en moles du composé
   (HOCH₂CH₂)₂NOC-CH₂- CH₂-CH₂-CON(CH₂CH₂OH)₂.

Selon un mode de réalisation le produit est différent du produit constitué de
- 20% en poids ou en moles du composé
   (HOCH₂CH₂)₂NOC-CH₂-CH₂-CON(CH₂CH₂OH)₂,
- 60% en poids ou en moles du composé
   (HOCH₂CH₂)₂NOC-CH₂- CH₂-CH₂-CON(CH₂CH₂OH)₂, et
- 20% en poids ou en moles du composé
   (HOCH₂CH₂)₂NOC-CH₂- CH₂-CH₂-CH₂-CON(CH₂CH₂OH)₂.

Selon un mode de réalisation le produit est différent du produit constitué de
- 75% en poids ou en moles du composé
   (HOCH₂CH₂)₂NOC -CH₂- CH₂-CH₂-CON(CH₂CH₂OH)₂, et
- 25% en poids ou en moles du composé
   (HOCH₂CH₂)₂NOC-CH₂- CH₂-CH₂-CH₂-CON(CH₂CH₂OH)₂.

On mentionne que le produit de l'invention peut comprendre au moins 10% en poids, de préférence au moins 50% en poids, de préférence au moins 75%, par exemple au moins 95%, des composés de formules (la), (Ib) et (Ic). A forte concentration le produit peut être un ingrédient ou une aide à des procédés destiné à être associé à d'autres ingrédients ou à être mis en oeuvre en présence d'autres composés chimiques. A faible concentration le produit peut être une composition ou une formulation comprenant d'autres ingrédients.

On mentionne que les composés (la), (Ib) et (Ic) peuvent être introduits séparément dans le produit. On préfère toutefois les introduire sous forme de mélange ou de pré-mélange. Selon un mode préférentiel le produit est issu d'un mélange réactionnel où les composés (la), (Ib) et (Ic) sont présents. Le produit peut notamment être un produit d'amidification ou de trans-amidification.

Le produit de l'invention présente avantageusement une température de fusion inférieure ou égale à 35°C, de préférence inférieure ou égale à 25°C, de préférence inférieure ou égale à 10°C, préférence inférieure ou égale à 0°C.

### Procédé

Le produit de l'invention peut être préparé par toute méthode appropriée. On préférera notamment mettre en oeuvre des réactions d'amidification ou de trans-amidification sur des diacides ou des diesters analogues, de préférence en mélanges analogues. De telles réactions sont connues de l'homme du métier.

Ainsi un procédé approprié de préparation d'un produit de l'invention peut être un procédé comprenant une étape d'amidification ou de trans-amidification par un composé de formule R²R³NH et/ou HNR⁴R⁵ d'un mélange d'au moins deux composés, de préférence les trois, choisis parmi les composés de formules (l'a), (I'b) et (I'c) suivantes:

R⁸OOC-A^{a}-COO R⁸ (I'a)

R⁸OOC-A^{b}-COO R⁸ (I'b)

R⁸OOC-A^{c}-COO R⁸ (I'c)

où R⁸ est un atome d'hydrogène ou un alkyle en C₁-C₆ de préférence un méthyle.

Les composés de formules (l'a), (I'b), et (I'c) sont considérés comme des diacides ou diesters analogues respectivement des composés diamides.

La réaction d'amidification ou de trans-amidification peut être menée de manière continue, semi-continue, ou discontinue ("batch"). Afin d'améliorer le taux de conversion de la réaction (taux de conversion de l'acide ou du diesters) et/ou de diminuer la quantité de produit non réagi dans une composition de matière et/ou d'augmenter la productivité, on peut notamment éliminer des sous produits de réaction au cours de cette dernière. On peut par exemple éliminer par évaporation ("stripping") des alcools formés au cours d'une réaction de trans-amidification. On note qu'on peut utiliser un très large excès d'amine, un sel d'amine ou tout autre moyen connu de l'homme du métier, par exemple décrit dans l'ouvrage "March's Advanced Organic Chemistry" de Michael B. Smith et Jerry March, 5e édition, John Wiley & Sons, pages 506-511.

La réaction peut être suivie d'étapes de filtration et/ou de purification par exemple par distillation.

On note que la réaction d'amidification ou de trans-amidification peut transiter par des intermédiaires activés tels les chlorures d'acides obtenus, par exemple, à partir des composés de formules (l'a), (I'b), (I'c) par réaction avec le chlorure de thionyle. La séparation de l'acide chlorhydrique, sous-produit au cours de ce type de réaction d'amidification, du milieu réactionnel par tout moyen adapté (formation de sel, distillation), constitue un moteur pour déplacer l'équilibre réactionnel vers la formation de l'amide recherché.

Les diacides ou diesters sous forme de mélanges peuvent notamment être des mélanges de diacides issus de la fabrication de l'acide adipique (mélange dit AGS) ou de diesters obtenus à partir de ces mélanges de diacides. Des mélanges de diesters sont notamment commercialisés sous la dénomination Rhodiasolv® RPDE.

### Utilisations - Formulations

Le produit l'invention peut notamment être utilisé comme solvant, co-solvant et/ou inhibiteur de cristallisation, comme agent plastifiant ou comme agent de coalescence.

Par co-solvant, on entend que d'autres solvants peuvent lui être associés. L'utilisation à titre de solvant ou de co-solvant comprend notamment l'utilisation pour dissoudre un composé dans une formulation, dans un milieu réactionnel, l'utilisation pour solubiliser totalement ou partiellement un produit à éliminer (dégraissage, décapage), et/ou pour faciliter de décollage de films de matières.

Le produit de l'invention peut notamment être utilisé, pour les fonctions indiquées ci-dessus ou pour d'autres, dans une formulation phytosanitaire, dans une formulation de nettoyage, dans une formulation de décapage, dans une formulation de dégraissage, dans une formulation de lubrifiants ou textiles, dans une formulation de revêtement, par exemple dans une formulation de peinture, dans une formulation de pigments ou encre, dans une formulation plastique.

Le produit peut par exemple être utilisé à titre d'agent de coalescence dans une formulation de peinture aqueuse.

Le produit peut notamment être utilisé comme solvant de résines par exemple dans l'industrie du revêtement de câbles ou dans l'industrie électronique, notamment comme solvant du PVDF.

Le produit peut notamment être utilisé comme solvant de nettoyage et/ou de décapage dans l'industrie électronique. Il peut notamment être utilisé dans des batteries au Lithium. Il peut notamment être utilisé sur des résines photorésistantes, des polymères, des cires des graisses, des huiles.

Le produit peut notamment être utilisé pour le nettoyage d'encres, par exemple lors de la production d'encres ou lors de l'utilisation d'encre en impression.

Le produit peut notamment être utilisé pour ne nettoyage de tamis ou d'autres outils mis en oeuvre dans des procédés de fabrication et/ou de recyclage de papier.

Le produit peut notamment être utilisé pour le nettoyage de bitumes ou de sables bitumineux ("tar sand" en anglais), par exemple sur les substrats revêtus, sur les outils utilisés pour appliquer ces matières, sur des vêtements souillés, sur des véhicules souillés.

Le produit peut notamment être utilisé pour le nettoyage d'engins volants comme des avions, des hélicoptères, des navettes spatiales.

Le produit peut notamment être utilisé comme agent de dégraissage sur des surfaces métalliques, par exemple des surfaces d'outils, d'objets manufacturés, de tôles, de moules, notamment en acier ou en aluminium ou en alliages de ces métaux.

Le produit peut notamment être utilisé comme solvant de nettoyage sur des surfaces dures ou des surfaces textiles.

Le produit peut notamment être utilisé comme solvant de décapage de peinture ou de résines, sur des surfaces d'outils, par exemple des moules de fonderie, sur des surfaces des sites industriels (sols, cloisons etc...). Les formulations de décapage de peintures peuvent notamment être des formulations à base aqueuse (le composé étant en mélange avec de l'eau) ou à base solvant (le composé étant alors le solvant ou un composé en mélange avec de l'eau).

Le produit peut notamment être utilisé comme agent plastifiant dans des formulations de polymères thermoplastiques.

Les formulations de nettoyage et/ou de dégraissage peuvent notamment être des formulations pour les soins ménagers, opérés dans les foyers ou dans les domaines publiques (hotels, bureaux, usines....). Il peut s'agir de formulations pour le nettoyage des surfaces dures comme les sols, les surfaces d'ameublement et d'équipement des cuisines et salles de bain, la vaisselle. Ces formulations peuvent également être utilisées dans la sphère industrielle pour dégraisser des produits manufacturés et/ou les nettoyés. De telles formulation peuvent notamment être utilisées pour nettoyer et/ou décaper des produits, des outils, des moules, des habits ou autres.

Le produit de l'invention peut notamment être utilisé dans des formulations phytosanitaires comprenant un produit actif solide. Plus de détails sont donnés ci-dessous, où le mot "solvant" peut désigner le produit de l'invention.

On mentionne qu'on peut associer au produit, ou introduire dans le produit des solvants ou co-solvants ou inhibiteurs de cristallisation autres, par les solvants de la famille des phosphates, phosphonates ou des oxydes de phosphines comme le TEBP, le TBP, le TEPO, le DBBP. On cite également les alkyldiméthyleamides où l'alkyl est en C₆-C₁₈, notamment ceux commercialisés sous la marque Genagen. On cite également les lactates d'esters, notamment ceux commercilisés sous la marque Purasolv. On cite également les esters méthyliques d'acides gras, notamment ceux commercialisés sous la marque Phytorobe. On cite également les diesters de diacides ("DiBasic Esters" en anglais), notamment ceux commercialisé par Rhodia sous les marques Rhodiasolv RPDE, et Rhodiasolv DIB. On cite également les coupes d'hydrocarbures, les amides cycliques comme la NMP, les lactones. On cite également les bis(dialkylamides) faisant l'objet du document WO 2008//074837.

### Utilisation détaillée dans le cadre de formulations phytosanitaires

La formulation phytosanitaire est généralement une formulation phytosanitaire concentrée comprenant un composé actif.

L'agriculture utilise de nombreuses matières actives telles que des fertilisants ou des pesticides, par exemple des insecticides, herbicides ou fongicides. On parle de produits phytosanitaires actifs (ou de matière active). Les produits phytosanitaires actifs sont généralement produits sous forme pure ou très concentrée. Ils doivent être utilisés sur les exploitations agricoles à de faibles concentrations. A cette fin, ils sont généralement formulés avec d'autres ingrédients afin de permettre une dilution en poids aisée par l'exploitant agricole. On parle de formulations phytosanitaires. La dilution opérée par l'exploitant agricole est généralement réalisée par mélange de la formulation phytosanitaire avec de l'eau.

Ainsi les formulations phytosanitaires doivent permettre une dilution en poids aisée par l'exploitant agricole, afin d'obtenir un produit dans lequel le produit phytosanitaire est correctement dispersé, par exemple sous forme de solution, d'émulsion, de suspension, ou de suspo-émulsion. Les formulations phytosanitaires permettent ainsi le transport d'un produit phytosanitaire sous forme relativement concentrée, un conditionnement aisé et/ou une manipulation aisée pour l'utilisateur final. Différents types de formulations phytosanitaires peuvent être utilisés selon les différents produits phytosanitaires. On cite par exemple les concentrés émulsionnables (Emulsifiable Concentrates «EC»), les émulsions concentrées (Emulsion in water "EW"), les microémulsions ("ME"), les poudres mouillables (Wettable Powders «WP»), les granulés dispersables dans l'eau (Water Dispersible Granules, «WDG»). Les formulations qu'il est possible d'utiliser dépendent de la forme physique du produit phytosanitaire (par exemple solide ou liquide), et de ses propriétés physico-chimiques en présence d'autres composés comme l'eau ou les solvants.

Après dilution en poids par l'exploitant agricole, par exemple par mélange avec de l'eau, le produit phytosanitaire peut se trouver sous différentes formes physiques: solution, dispersion de particules solides, dispersion de gouttelettes du produit, gouttelettes de solvant dans lequel le produit est dissous... Les formulations phytosanitaires comprennent généralement des composés permettant d'obtenir ces formes physiques. Il peut par exemple s'agir de tensioactifs, de solvants, de supports minéraux, et/ou de dispersants. Bien souvent ces composés n'ont pas un caractère actif, mais un caractère d'intermédiaire d'aide à la formulation. Les formulations phytosanitaires peuvent notamment être sous forme liquide, ou sous forme solide.

Afin de préparer des formulations phytosanitaires de produits phytosanitaires actifs solides, il est connu de solubiliser le produit dans un solvant. La formulation phytosanitaire comprend ainsi une solution du produit dans le solvant. La formulation peut être sous forme solide, par exemple sous forme de poudre mouillable (WP) où la solution imbibe un support inorganique, par exemple du kaolin et/ou de la silice. La formulation peut alternativement être sous forme liquide, par exemple sous forme de concentré émulsionnable (EC) présentant une seule phase liquide limpide comprenant le solvant et le produit en solution, pouvant former une émulsion par ajout d'eau, sans agitation ou avec une faible agitation. Elle peut aussi être ou sous forme d'une émulsion concentrée (EW), trouble, dont la phase dispersée dans l'eau comprend le solvant et le produit en solution dans le solvant. Elle peut aussi être forme d'une microémulsion (ME), limpide, dont la phase dispersée dans l'eau comprend le solvant et le produit en solution dans le solvant.

Certains actifs phytosanitaires solides sont souvent difficiles à formuler. Par exemple le tebuconazole est un fongicide particulièrement efficace, et d'utilisation répandue, pour la culture du soja notamment. Pour certains actifs phytosanitaires, il est difficile de réaliser des formulations concentrées, faciles à diluer pour l'exploitant agricole, stables, et sans inconvénients (avérés ou perçus) substantiels en matière de sécurité, de toxicité et/ou d'eco-toxicité. Pour certains actifs, il est difficile de formuler à des concentrations relativement élevées, avec une stabilité suffisante. En particulier il est nécessaire d'éviter l'apparition de cristaux en particulier à basse température et/ou lors de la dilution et/ou lors du stockage à température élevée de la composition diluée. Les cristaux peuvent avoir des effets négatifs, notamment boucher les filtres des dispositifs utiliser pour répandre la composition diluée, boucher les dispositifs de pulvérisation, diminuer l'activité globale de la formulation, créer des problèmes inutiles de filières de déchets pour éliminer les cristaux, et/ou provoquer une mauvaise répartition du produit actif sur le champ agricole.

Les formulations comprenant le solvant présentent notamment:
- une solubilisation de quantités importantes d'actifs,
- une absence de cristallisation, même des conditions exigeantes,
- une bonne activité biologique pouvant être due à une bonne solvatation, et/ou
- un profile de sécurité, toxicologie et/ou eco-toxicologie perçu comme favorable.

La formulation phytosanitaire peut en outre être une formulation phytosanitaire concentrée comprenant:
a) un produit phytosanitaire actif,
b) le solvant (produit de l'invention)
c) éventuellement au moins agent émulsifiant, de préférence un tensioactif, et
d) éventuellement de l'eau.

### Produit phytosanitaire actif a)

Des produits phytosanitaires actifs, notamment des produits non solubles dans l'eau et solides sont connus de l'homme du métier. Le produit phytosanitaire actif peut notamment être un herbicide, un insecticide, un acaricide, un fongicide, ou un agent d'élimination des rongeurs ("rodenticide" en anglais) par exemple un raticide.

A titre d'exemples non limitatifs de matières actives convenables, on peut citer entre autres l'Amétryne, le Diuron, le Linuron, le Chlortoluron, l'Isoproturon, le Nicosulfuron, le Metamitron, le Diazinon, l'Aclonifen, l'Atrazine, le Chlorothalonil, le Bromoxynil, le Bromoxynil heptanoate, le Bromoxynil octanoate, le Mancozeb, la Manèbe, le Zineb, la Phenmédipham, le Propanyl, la série des phénoxyphénoxy, la série des hétéroaryloxyphénoxy, le CMPP, le MCPA, le 2,4-D, la Simazine, les produits actifs de la série des imidazolinones, la famille des organophosphorés, avec notamment l'Azinphos-éthyl, l'Azinphos-méthyl, l'Alachlore, le Chlorpyriphos, le Diclofop-méthyl, le Fénoxaprop-p-éthyl, le Méthoxychlore, la Cyperméthrine, le Fenoxycarbe, le cymoxanil, le chlorothalonyl, Ikes insecticides neonicotinoides, la famille des fongicide triazoles tels que l'azaconazole, bromuconazole, cyproconazole, difenoconazole, diniconazole, epoxyconazole, fenbuconazole, flusilazole, myclobutanyl, tebuconazole, triadimefon, triadimenol, des strobilurines telles que la pyraclostrobine, la picoxystrobine, l'azoxystrobine, la famoxadone, le kresoxym-methyl et la trifloxystrobine, les solfonylurées telles que le bensulfuron-methyl, le chlorimuron-ethyl, le chlorsulfuron, le metsulfuron-methyl, le nicosulfuron, le sulfomethuron-methyl, le triasulfuron, le tribenuron-methyl.

On choisi parmi cette liste les produits non-hydrosolubles.

On peut notamment mettre en oeuvre les produits phytosanitaires actifs suivants: Alachlor, Chlorpyrifos, alpha-cyperméthrine, Phenmedipham, Propanil, Pendimethalin, Triadimenol, Trifluralin, Oxyfluorfen, Dimethoate, Imidacloprid, Proxopur, Benomyl, Deltamethrine, Fenvalerate, Abamectin, Amicarbazone, Bifenthrin, Carbosulfan, Cyfluthrin, Difenconazole, Ethofenprox, Fenoxaprop-ethyl, Fipronil, Fenvalerate, Fluazifop-p-butyl, Flufenouron, Hexazinone, Lambda-cyalothrin, Methomyl, Permethrin, Prochloraz, Propiconazole, Tebuconazole

Ces produits et dénominations sont connus de l'homme du métier. On peut associer plusieurs produits phytosanitaires actifs.

### Agent émulsifiant c)

La formulation phytosanitaire peut comprendre un agent émulsifiant, typiquement et de préférence un tensioactif. Les agents émulsifiants sont des agents destinés à faciliter la mise en émulsion ou la dispersion après mise en présence de la formulation avec de l'eau, et/ou à stabiliser (dans le temps et/ou en température) l'émulsion ou la dispersion, par exemple en évitant une sédimentation.
Les tensioactifs sont des composés connus, qui présentent une masse molaire généralement relativement faible, par exemple inférieure à 1000 g/mol. Le tensioactif peut être un tensioactif anionique sous forme salifiée ou acide, non ionique de préférence polyalcoxylé, cationique, amphotère (terme incluant aussi les tensioactifs zwitterioniques). Il peut s'agir d'un mélange ou d'une association de ces tensioactifs.

A titre d'exemples de tensioactifs anioniques, on peut citer, sans intention de s'y limiter:
- les acides alkylsulfoniques, les acides arylsulfoniques, éventuellement substitués par un ou plusieurs groupements hydrocarbonés, et dont la fonction acide est partiellement ou totalement salifiée, comme les acides alkylsulfoniques en C₈-C₅₀, plus particulièrement en C₈-C₃₀, de préférence en C₁₀-C₂₂, les acides benzènesulfoniques, les acides naphtalènesulfoniques, substitués par un à trois groupements alkyles en C₁-C₃₀, de préférence en C₄-C₁₆, et/ou alcényles en C₂-C₃₀, de préférence en C₄-C₁₆.
- les mono- ou diesters d'acides alkylsulfosucciniques, dont la partie alkyle, linéaire ou ramifiée, éventuellement substituée par un ou plusieurs groupements hydroxylés et/ou alcoxylés, linéaires ou ramifiés en C₂-C₄ (de préférence éthoxylés, propoxylés, éthopropoxylés).
- les esters phosphates choisis plus particulièrement parmi ceux comprenant au moins un groupement hydrocarboné saturé, insaturé ou aromatique, linéaire ou ramifié, comprenant 8 à 40 atomes de carbone, de préférence 10 à 30, éventuellement substitués par au moins un groupement alcoxylé (éthoxylé, propoxylé, éthopropoxylé). En outre, ils comprennent au moins un groupe ester phosphate, mono- ou diestérifié de telle sorte que l'on puisse avoir un ou deux groupes acides libres ou partiellement ou totalement salifiés. Les esters phosphates préférés sont du type des mono- et diesters de l'acide phosphorique et de mono-, di- ou tristyrylphénol alcoxylé (éthoxylé et/ou propoxylé), ou de mono-, di- ou trialkylphénol alcoxylé (éthoxylé et/ou propoxylé), éventuellement substitué par un à quatre groupements alkyles ; de l'acide phosphorique et d'un alcool en C₈-C₃₀, de préférence en C₁₀-C₂₂ alcoxylé (éthoxylé ou éthopropoxylé); de l'acide phosphorique et d'un alcool en C₈-C₂₂, de préférence en C₁₀-C₂₂, non alcoxylé.
- les esters sulfates obtenus à partir d'alcools saturés, ou aromatiques, éventuellement substitués par un ou plusieurs groupements alcoxylés (éthoxylés, propoxylés, éthopropoxylés), et pour lesquels les fonctions sulfates se présentent sous la forme acide libre, ou partiellement ou totalement neutralisées. A titre d'exemple, on peut citer les esters sulfates obtenus plus particulièrement à partir d'alcools en C₈-C₂₀, saturés ou insaturés, pouvant comprendre 1 à 8 motifs alcoxylés (éthoxylés, propoxylés, éthopropoxylés) ; les esters sulfates obtenus à partir de phénol polyalcoxylé, substitués par 1 à 3 groupements hydroxycarbonés en C₂-C₃₀, saturés ou insaturés, et dans lesquels le nombre de motifs alcoxylés est compris entre 2 et 40 ; les esters sulfates obtenus à partir de mono-, di- ou tristyrylphénol polyalcoxylés dans lesquels le nombre de motifs alcoxylés varie de 2 à 40.

Les tensioactifs anioniques peuvent être sous forme acide (il sont potentiellement anioniques), ou sous une forme partiellement ou totalement salifiée, avec un contre-ion. Le contre-ion peut être un métal alcalin, tel que le sodium ou le potassium, un alcalino-terreux, tel que le calcium, ou encore un ion ammonium de formule N(R)₄⁺ dans laquelle R, identiques ou différents, représentent un atome d'hydrogène ou un radical alkyle en C₁-C₄ éventuellement substitué par un atome d'oxygène.

A titres d'exemples de tensioactifs non ioniques, on peut citer, sans intention de s'y limiter:
- les phénols polyalcoxylés (éthoxylés, propoxylés, éthopropoxylés) substitués par au moins un radical alkyle en C₄-C₂₀, de préférence en C₄-C₁₂, ou substitués par au moins un radical alkylaryle dont la partie alkyle est en C₁-C₆. Plus particulièrement, le nombre total de motifs alcoxylés est compris entre 2 et 100. A titre d'exemple, on peut citer les mono-, di- ou tri (phényléthyl) phénols polyalcoxylés, ou les nonylphénols polyalcoxylés. Parmi les di- ou tristyrylphenols éthoxylés et/ou propoxylés, sulfatés et/ou phosphatés, on peut citer, le di-(phényl-1 éthyl)phénol éthoxylé, contenant 10 motifs oxyéthylénés, le di-(phényl-1 éthyl)phénol éthoxylé, contenant 7 motifs oxyéthylénés, le di-(phényl-1 éthyl)phénol éthoxylé sulfaté, contenant 7 motifs oxyéthylénés, le tri-(phényl-1 éthyl)phénol éthoxylé, contenant 8 motifs oxyéthylénés, le tri-(phényl-1 éthyl)phénol éthoxylé, contenant 16 motifs oxyéthylénés, le tri-(phényl-1 éthyl)phénol éthoxylé sulfaté, contenant 16 motifs oxyéthylénés, le tri-(phényl-1 éthyl)phénol éthoxylé, contenant 20 motifs oxyéthylénés, le tri-(phényl-1 éthyl)phénol éthoxylé phosphaté, contenant 16 motifs oxyéthylénés.
- les alcools ou les acides gras en C₆-C₂₂, polyalcoxylés (éthoxylés, propoxylés, éthopropoxylés). Le nombre des motifs alcoxylés est compris entre 1 et 60. Le terme acide gras éthoxylé inclut aussi bien les produits obtenus par éthoxylation d'un acide gras par l'oxyde d'éthylène que ceux obtenus par estérification d'un acide gras par un polyéthylèneglycol.
- les triglycérides polyalcoxylés (éthoxylés, propoxylés, éthopropoxylés) d'origine végétale ou animale. Ainsi conviennent les triglycérides issus du saindoux, du suif, de l'huile d'arachide, de l'huile de beurre, de l'huile de graine de coton, de l'huile de lin, de l'huile d'olive, de l'huile de palme, de l'huile de pépins de raisin, de l'huile de poisson, de l'huile de soja, de l'huile de ricin, de l'huile de colza, de l'huile de coprah, de l'huile de noix de coco, et comprenant un nombre total de motifs alcoxylés compris entre 1 et 60. Le terme triglycéride éthoxylé vise aussi bien les produits obtenus par éthoxylation d'un triglycéride par l'oxyde d'éthylène que ceux obtenus par trans-estérification d'un triglycéride par un polyéthylèneglycol.
- les esters de sorbitan éventuellement polyalcoxylés (éthoxylés, propoxylés, éthopropoxylés), plus particulièrement les esters de sorbitol cyclisé d'acides gras de C₁₀ à C₂₀ comme l'acide laurique, l'acide stéarique ou l'acide oléique, et comprenant un nombre total de motifs alcoxylés compris entre 2 et 50.

Des émulsifiants utiles sont notamment les produits suivants, tous commercialisés par Rhodia:
- Soprophor® TSP/724: tensioactif à base de tristyrylphonol éthopropoxylé
- Soprophor® 796/O: tensioactif à base de tristyrylphonol éthopropoxylé
- Soprophor® CY 8: tensioactif à base de tristyrylphonol éthoxylé
- Soprophor® BSU: tensioactif à base de tristyrylphonol éthoxylé
- Alkamuls® RC: tensioactif à base d'huile de ricin éthoxylée
- Alkamuls® OR/36: tensioactif à base d'huile de ricin éthoxylée
- Alkamuls® T/20: tensioactif à base d'un ester de sorbitan

La formulation comprend avantageusement au moins 4%, de préférence au moins 5%, de préférence au moins 8%, en poids de matière sèche, d'au moins un tensioactif c).

On mentionne que le solvant peut être associé à un tensioactif aromatique et/ou non aromatique.

### Autres détails quant à la formulation phytosanitaire

La formulation phytosanitaire, concentrée, ne comprend de préférence pas des quantités importantes d'eau. Typiquement la teneur en eau est inférieure à 50% en poids, avantageusement inférieure à 25% en poids. Elle sera généralement inférieure à 10% en poids.

La formulation est de préférence un formulation liquide, par exemple sous forme d'un concentré emulsifiable (EC), d'une émulsion concentrée (EW) ou d'une micorémulsion (ME). Dans ce cas elle comprend de préférence moins de 500 g/L d'eau, plus préférablement moins de 250 g/L. Elle sera généralement inférieure à 100 g/L.

Les formulations peuvent avantageusement comprendre:
a) de 4 à 60%, de préférence de 10 à 50%, du produit phytosanitaire, en poids de matière active,
b) de 10 à 92%, de préférence de 20 à 80%, du solvant, en poids,
c) de 4 à 60%, de préférence de 5 à 50%, de préférence de 8 à 25%, en poids de matière sèche, d'un émulsifiant, de préférence d'un tensioactif,
d) de 0 à 10% en poids d'eau.

Il n'est pas exclu de réaliser des formulations solides, par exemple des formulations dans lesquelles un liquide comprenant le produit phytosanitaire solubilisé dans le solvant, est supporté par un minéral et/ou dispersé dans une matrice solide.

La formulation peut bien entendu comprendre d'autres ingrédients (ou "autres additifs") que le produit phytosanitaire actif, le(s) solvant(s), le(s) agent(s) émulsifiant(s) optionnel(s) et l'eau optionnelle. Elle peut notamment comprendre des agents de modification de la viscosité, des agents antimousse, notamment des antimousse siliconnés, des agents anti-rebond, des agents anti-lessivage, des charges inertes, notamment des charges minérales, des agents anti-gel...

Notamment les formulations peuvent comprendre des additifs, dits autres additifs, ne rentrant pas dans la définition des produits a), b), ou c), comme:
- d'autres solvants, généralement en faible quantité, par exemple en quantité inférieure à la quantité de produit de l'invention. A titre d'autres solvants on cite notamment les solvants de la famille des phosphates, phosphonates ou des oxydes de phosphines comme le TEBP, le TBP, le TEPO, le DBBP. On cite également les alkyldiméthyleamides où l'alkyl est en C₆-C₁₈, notamment ceux commercialisés sous la marque Genagen. On cite également les lactates d'esters, notamment ceux commercilisés sous la marque Purasolv. On cite également les esters méthyliques d'acides gras, notamment ceux commercialisés sous la marque Phytorobe. On cite également les diesters de diacides ("DiBasic Esters" en anglais), notamment ceux commercialisé par Rhodia sous les marques Rhodiasolv RPDE, et Rhodiasolv DIB. On cite également les coupes d'hydrocarbures, les amides cycliques comme la NMP, les lactones. On cite également les bis(dialkylamides) faisant l'objet du document WO 2008//074837.
- des inhibiteurs de cristallisation. Il peut s'agir des solvants mentionnés ci-dessus. Il peut également s'agir d'acides gras ou d'alcools gras non polyalkoxylés. On cite par exemple le produit Alkamuls® OL700 commercialisé par Rhodia.

Des procédés classiques de préparation de formulations phytosanitaires ou de mélanges de solvants peuvent être mis en oeuvre. On peut opérer par simple mélange des constituants.

La formulation phytosanitaire concentrée est destinée à être répandue sur un champ cultivé ou à cultiver, par exemple un champ de soja, le plus souvent après dilution dans de l'eau, pour obtenir une composition diluée. La dilution est généralement opérée par l'exploitant agricole, directement dans un réservoir ("tank-mix"), par exemple dans le réservoir d'un dispositif destiné à répandre la composition. Il n'est pas exclu que l'exploitant ajoute d'autres produits phytosanitaires, par exemple des fongicides, herbicides, pesticides, insecticides, des fertilisants. Ainsi, la formulation peut être utilisée pour préparer une composition diluée dans l'eau du produit phytosanitaire actif, par mélange d'au moins une part en poids de formulation concentrée avec au moins 10 parts d'eau, de préférence moins de 1000 parts. Les taux de dilution et les quantités à appliquer sur le champ dépendent généralement du produit phytosanitaire et de la dose souhaitable pour traiter le champ; cela peut être déterminé par l'exploitant agricole.

D'autres détails ou avantages pourront apparaître au vu des exemples qui suivent, sans caractère limitatif.

### EXEMPLES

On prépare des mélanges de diamides à partir de mélanges de diacide. On met en oeuvre pour la préparation les procédures générales suivantes (dans les formules A représente un mélange de A^{a} = éthylène, A^{b} = n, propylène, A^{c} = n-butylène).

### Procédure A: Formation de dichlorures d'acide (en mélange)

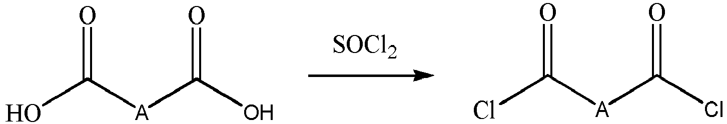

Le mélange de diacides et le chlorure de thionyle sont mélangés à température ambiante. Le mélange réactionnel peut être chauffé au reflux pour compléter la réaction. Les espèces volatiles sont retirées par distillation sous pression réduite pour obtenir le produit brut qui est typiquement utilisé tel quel sans autre forme de purification.

### Procédure B: Formation de diamide (en mélange) par réaction avec une dialkylamine

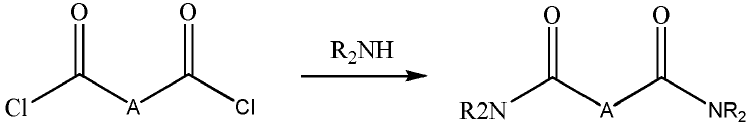

Du toluène et de la triéthylamine sont chargés et refroidis a -10°C. La dialkylamine est alors chargée sous forme liquide. Le dichlorure d'acide (en mélange), dilué dans du toluène, est alors ajouté lentement tout en maintenant la température en dessous de +10°C. Le mélange est alors agité pendant deux heures à température ambiante. Les sels formés durant la réaction sont alors filtrés et lavés avec de l'acétate d'éthyle. Le filtrat ainsi obtenu est évaporé à l'évaporateur rotatif pour obtenir le brut de réaction. Le produit final est finalement obtenu après une distillation sous pression réduite.

### Exemple 1.1 Préparation d'un produit comprenant un mélange des composés suivants:

Me₂NOC-CH₂-CH₂-CONMe₂
Me₂NOC-CH₂-CH₂-CH₂-CONMe₂, et
Me₂NOC-CH₂-CH₂- CH₂-CH₂-CONMe₂

### Etape 1

### Matière premières

| Mélange d'acide adipique, d'acide gutarique et d'acide succinique | Chlorure de thionyle |
|---|---|
| 500g; 3,82 mol; Mw: moyenne 132 | 1127ml; 15,30mol; Mw: 118,97; 4eq |

Le dichlorure d'acide (en mélange) est obtenu par la Procédure A.
Produit brut = 631g

### Etape 2

### Matière premières

| Dichlorure d'acide (en mélange) | DMA (diméthylamine) | TEA | Toluène |
|---|---|---|---|
| 609g; 3,64 mol; 1 eq. Mw: moyenne 169 | 404ml; 8,96 mol; 1,23eq; Mw: 45,08 | 1280ml; 9,11 mol; 1,25eq; Mw:101,2 | 2000+1000ml |

Le dialkyl diamide (en mélange) est obtenu par la Procédure B.
Produit brut = 729g (liquide marron)
Produit final = 574g (liquide légèrement jaune)
Analyse CG (surface) > 98% (somme des isomères)

Le produit obtenu est analysé par chromatographie en phase gazeuse. L'analyse indique (en surface de pic):

| | |
|---|---|
| Me₂NOC-CH₂-CH₂-CONMe₂ | 13,49% |
| Me₂NOC-CH₂-CH₂-CH₂-CONMe₂ | 71,87% |
| Me₂NOC-CH₂-CH₂- CH₂-CH₂-CONMe₂ | 13,31% |

On observe le produit à différentes températures. On constate qu'il est liquide à des températures de 35°C, de 30°C, de 25°C, de 20°C, de 10°C. On constate également qu'il est exempt de cristaux à ces températures.

### Exemple 1.2 Préparation d'un produit comprenant un mélange des composés suivants:

Et₂NOC-CH₂-CH₂-CONEt₂
Et₂NOC-CH₂-CH₂-CH₂-CONEt₂, et
Et₂NOC-CH₂-CH₂- CH₂-CH₂-CONEt₂

### Etape 1

### Matière premières

| Mélange d'acide adipique, d'acide gutarique et d'acide succinique | Chlorure de thionyle |
|---|---|
| 500g; 3,82 mol; Mw: moyenne 132 | 1127ml; 15,30mol; Mw: 118,97; 4eq |

Le dichlorure d'acide (en mélange) est obtenu par la Procédure A.
Produit brut = 615g

### Etape 2

### Matière premières

| Dichlorure d'acide (en mélange) | DEA (diethylamine) | TEA | Toluène |
|---|---|---|---|
| 609g; 3,64 mol; 1 eq. Mw: moyenne 169 | 955ml; 9,20 mol; 1,25eq; Mw: 73,14 | 1292ml; 9,20 mol; 1,25eq; Mw:101,2 | 2000+1000ml |

Le dialkyl diamide (en mélange) est obtenu par la Procédure B.
Produit brut = 1068 g (liquide marron)
Produit final = 700 g (liquide légèrement jaune)
Analyse CG (surface) > 96% (somme des isomères)

Le produit obtenu est analysé par chromatographie en phase gazeuse. L'analyse indique (en surface de pic):

| | |
|---|---|
| Et₂NOC-CH₂-CH₂-CONEt₂ | 13,09% |
| Et₂NOC-CH₂-CH₂-CH₂-CONEt₂ | 70.60% |
| Et₂NOC-CH₂-CH₂- CH₂-CH₂-CONEt₂ | 12,71% |

On observe le produit à différentes températures. On constate qu'il est liquide à des températures de 35°C, de 30°C, de 25°C, de 20°C, de 10°C, de 0°C, de -10°C. On constate également qu'il est exempt de cristaux à ces températures.

### Exemples 2 à 4 - Utilisations comme solvants - Formulations phytosanitaires

Par mélange des ingrédients, on prépare des formulations de divers actifs phytosanitaires, de type concentré émulsionnable (EC).
Les formulations comprennent:
- l'actif, en quantité en poids (de matière active) indiquée dans le tableau ci-dessous,
- 10% en poids de tensioactif Alkamuls® RC, commercialisé par Rhodia
- et, comme solvant, le reste de composé des exemples.

Les exemples 2 sont des exemples comparatifs où est utilisé comme solvant le produit Rhodiasolv® ADMA10, Rhodia (zone Asie Pacifique): Solvant alkyldiméthylamide.

On effectue les tests suivants:
- Observation visuelle à 25°C - On note l'aspect de la formulation et on repère éventuellement la présence de cristaux
- Observation visuelle à 0°C - On place la formulation pendant 7 jours à 0°C et on note l'aspect de la formulation et on repère éventuellement la présence de cristaux (test CIPAC MT39)
- Observation visuelle à 0°C avec nucléation: On introduit un cristal de la matière active dans la formulation ayant passé 7 jours à 0°C pour nucléation, et on place à nouveau la formulation pendant 7 jours à 0°C. On note l'aspect de la formulation et on repère éventuellement la présence de cristaux.

| *Exemple* | *Solvant* | *Actif* | *Apparence à 25°C* | *Apparence à 0°C* | *Apparence à 0°C avec nucléation* |
|---|---|---|---|---|---|
| 2.1C | Rhodiasolv® ADMA 10 | Alachlor - 48% | Limpide | Cristaux | Cristaux |
| 2.6C | Rhodiasolv® ADMA 10 | Pendimethalin - 33% | Limpide | Cristaux | Cristaux |
| 2.8C | Rhodiasolv® ADMA 10 | Triadimenol - 23% | Limpide | Limpide | Cristaux |
| 2.10C | Rhodiasolv® ADMA 10 | Difenconazole - 25% | Limpide | Limpide | Cristaux |
| 2.11C | Rhodiasolv® ADMA 10 | Imidacloprid - 20% | Non soluble | Non soluble | Non soluble |
| 2.12C | Rhodiasolv® ADMA 10 | Dimethoate - 40% | Trouble | Trouble | Cristaux |
| 2.13C | Rhodiasolv® ADMA 10 | Oxyfluorfen - 22% | Limpide | Limpide | Cristaux |
| 2.14C | Rhodiasolv® ADMA 10 | Propoxur - 20% | Limpide | Limpide | Cristaux |
| 3.1 | Exemple 1.1 | Alachlor - 48% | Limpide | Limpide | Limpide |
| 3.2 | Exemple 1.1 | Chlorpyrifos - 40% | Limpide | Limpide | Limpide |
| 3.5 | Exemple 1.1 | Propanil - 36% | Limpide | Limpide | Limpide |
| 3.6 | Exemple 1.1 | Pendimethalin - 33% | Limpide | Limpide | Cristaux |
| 3.11 | Exemple 1.1 | Imidacloprid - 20% | Limpide | Limpide | Limpide |
| 3.12 | Exemple 1.1 | Dimethoate - 40% | Limpide | Limpide | Cristaux |
| 4.1 | Exemple 1.2 | Alachlor - 48% | Limpide | Limpide | Limpide |
| 4.2 | Exemple 1.2 | Chlorpyrifos - 40% | Limpide | Limpide | Limpide |
| 4.3 | Exemple 1.2 | Alpha-Cypermethrine - 10% | Limpide | Limpide | Limpide |
| 4.5 | Exemple 1.2 | Propanil - 36% | Limpide | Limpide | Limpide |
| 4.7 | Exemple 1.2 | Tebuconazol - 23% | Limpide | Limpide | Limpide |
| 4.8 | Exemple 1.2 | Triadimenol - 23% | Limpide | Limpide | Limpide |
| 4.10 | Exemple 1.2 | Difenconazole - 25% | Limpide | Limpide | Limpide |
| 4.13 | Exemple 1.2 | Oxyfluorfen - 22% | Limpide | Limpide | Limpide |
| 4.14 | Exemple 1.2 | Propoxur - 20% | Limpide | Limpide | Limpide |

### Exemple 5 - Formulation d'imidacloprid

On prépare la formulation EC suivante:

| | |
|---|---|
| Imidacloprid tech.98% | 204 g/L |
| Solvant de l'exemple 1.1 | 856 g/L |
| Soprophor® 769/P (tensioactif, Rhodia) | 150 g/L |

On évalue les propriétés de la formulation après la préparation:
- Densité à 20°C: 1,120
- pH (Méthode Cipoac MT 75): 3,8
- Emulsification (test Cipac, à 0,5% de concentration à 30°C, après 2 heures

| | | |
|---|---|---|
| A | D | C |
| 0t | 0 | 0 |

- Apparence à 0°C: Solution Limpide
- Apparence à 54°C: Solution limpide

On évalue les propriétés de la formulation après 14 jours à 54°C:
- pH (Méthode Cipoac MT 75): 3,0
- Emulsification (test Cipac, à 0,5% de concentration à 30°C, après 2 heures

| | | |
|---|---|---|
| A | D | C |
| 0t | 0 | 0 |

## Revendications

1. Produit comprenant au moins deux composés diamides choisis parmi les composés diamides de formules (la), (Ib) et (Ic) suivantes:
R²R³NOC-A^{a}-CONR⁴R⁵ (Ia)
R²R³NOC-A^{b}-CONR⁴R⁵ (Ib)
R²R³NOC-A^{c}-CONR⁴R⁵ (Ic)
où:
- R², R³, R⁴ et R⁵, identiques ou différents, sont choisis parmi les groupes méthyle, éthyle, propyle (n-propyl), isopropyle, n-butyle, isobutyle, n-pentyle, amyle, isoamyle, hexyle, cyclohexyle, hydroxyéthyle, morpholine, pipérazine ou pipéridine ;
- A^{a} est le groupe de formule suivante -CH₂-CH₂- (éthylène)
- A^{b} est le groupe de formule suivante -CH₂-CH₂-CH₂- (n-propylène), et
- A^{c} est le groupe de formule suivante -CH₂-CH₂-CH₂-CH₂- (n-butylène).

2. Produit selon l'une des revendications précédentes, **caractérisé en ce qu'**il comprend les trois composés de formules (la), (Ib) et (Ic).

3. Produit selon l'une des revendications précédentes, **caractérisé en ce qu'**il comprend par rapport au nombre total de moles de diamides de formules (la), (Ib), et (Ic):
- de 1 à 20%, de préférence de 5 à 15%, en moles, de diamide de formule (Ic)
- de 45 à 75%, de préférence de 55 à 65%, en moles, de diamide de formule (Ib), et
- de 15 à 45%, de préférence de 20 à 33%, en moles, de diamide de formule (la).

4. Produit selon l'une des revendications précédentes, **caractérisé en ce que**:
- le composé de formule (la) est le composé Me₂NOC-CH₂-CH₂-CONMe₂
- le composé de formule (Ib) est le composé Me₂NOC-CH₂-CH₂-CH₂-CONMe₂, et
- le composé de formule (Ic) est le composé Me₂NOC-CH₂-CH₂- CH₂-CH₂-CONMe₂.

5. Produit selon l'une des revendications 1 à 3, **caractérisé en ce que**:
- le composé de formule (la) est le composé Et₂NOC-CH₂-CH₂-CONEt₂
- le composé de formule (Ib) est le composé Et₂NOC-CH₂-CH₂-CH₂-CONEt₂, et
- le composé de formule (Ic) est le composé Et₂NOC-CH₂-CH₂- CH₂-CH₂-CONEt₂.

6. Produit selon l'une des revendications précédentes, **caractérisé en ce qu'**il comprend au moins 10% en poids, de préférence au moins 50% en poids, des composés de formules (la), (Ib) et (Ic).

7. Produit selon la revendication 6, **caractérisé en ce que** c'est un produit d'amidification ou de trans-amidification.

8. Produit selon l'une des revendications précédentes, **caractérisé en ce qu'**il présente une température de fusion inférieure ou égale à 35°C, de préférence inférieure ou égale à 25°C, de préférence inférieure ou égale à 10°C, préférence inférieure ou égale à 0°C.

9. Procédé de préparation d'un produit selon l'une des revendications précédentes, comprenant une étape d'amidification ou de trans-amidification par un composé de formule R²R³NH et/ou HNR⁴R⁵ d'un mélange d'au moins deux composés choisis parmi les composés de formules (l'a), (I'b) et (I'c) suivantes:
R⁸OOC-A^{a}-COO R⁸ (I'a)
R⁸OOC-A^{b}-COO R⁸ (I'b)
R⁸OOC-A^{c}-COO R⁸ (I'c)
où R⁸ est un atome d'hydrogène ou un alkyle en C₁-C₆ de préférence un méthyle.

10. Utilisation comme solvant, plastifiant, ou agent de coalescence d'un produit tel que défini dans l'une des revendications 1 à 8.

11. Utilisation du produit tel que défini dans l'une des revendications 1 à 8, dans une formulation phytosanitaire, dans une formulation du nettoyage, dans une formulation de décapage, dans une formulation de dégraissage, dans une formulation de lubrifiants, dans une formulation de revêtement, dans une formulation de pigments ou encre, ou dans une formulation plastique.

## Patentansprüche

1. Erzeugnis umfassend mindestens zwei Diamid-Verbindungen ausgewählt aus den Diamid-Verbindungen der folgenden Formeln (Ia), (Ib) und (Ic):
R²R³NOC-A^{a}-CONR⁴R⁵ (Ia)
R²R³NOC-A^{b}-CONR⁴R⁵ (Ib)
R²R³NOC-A^{c}-CONR⁴R⁵ (Ic)
wobei:
- R², R³, R⁴ und R⁵, identisch oder verschieden, ausgewählt sind aus Methyl-, Ethyl-, Propyl- (n-Propyl-), Isopropyl-, n-Butyl-, Isobutyl-, n-Pentyl, Amyl-, Isoamyl-, Hexyl-, Cyclohexyl-, Hydroxyethyl-, Morpholin-, Piperazin- oder Piperidingruppen;
- A^{a} die Gruppe der folgenden Formel -CH₂-CH₂- (Ethylen) ist,
- A^{b} die Gruppe der folgenden Formel -CH₂-CH₂-CH₂- (n-Propylen) ist, und
- A^{c} die Gruppe der folgenden Formel -CH₂-CH₂-CH₂-CH₂- (n-Butylen) ist.

2. Erzeugnis gemäß einem der vorangehenden Ansprüche, **gekennzeichnet dadurch, dass** es die drei Verbindungen der Formeln (Ia), (Ib) und (Ic) umfasst.

3. Erzeugnis gemäß einem der vorangehenden Ansprüche, **gekennzeichnet dadurch, dass** es im Verhältnis zur absoluten Molzahl der Diamide der Formeln (Ia), (Ib) und (Ic):
- von 1 bis 20 Mol-%, vorzugsweise von 5 bis 15 Mol-% an Diamid der Formel (Ic),
- von 45 bis 75 Mol-%, vorzugsweise von 55 bis 65 Mol-% an Diamid der Formel (Ib) und
- von 15 bis 45 Mol-%, vorzugsweise von 20 bis 33 Mol-% an Diamid der Formel (Ia)
umfasst.

4. Erzeugnis gemäß einem der vorangehenden Ansprüche, **gekennzeichnet dadurch, dass**:
- die Verbindung der Formel (Ia) die Verbindung Me₂NOC-CH₂-CH₂-CONMe₂ ist,
- die Verbindung der Formel (Ib) die Verbindung Me₂NOC-CH₂-CH₂-CH₂-CONMe₂ ist, und
- die Verbindung der Formel (Ic) die Verbindung Me₂NOC-CH₂-CH₂-CH₂-CH₂-CONMe₂ ist.

5. Erzeugnis gemäß einem der Ansprüche 1 bis 3, **gekennzeichnet dadurch, dass**:
- die Verbindung der Formel (Ia) die Verbindung Et₂NOC-CH₂-CH₂-CONEt₂ ist,
- die Verbindung der Formel (Ib) die Verbindung Et₂NOC-CH₂-CH₂-CH₂-CONEt₂ ist, und
- die Verbindung der Formel (Ic) die Verbindung Et₂NOC-CH₂-CH₂-CH₂-CH₂-CONEt₂ ist.

6. Erzeugnis gemäß einem der vorangehenden Ansprüche, **gekennzeichnet dadurch, dass** es mindestens 10 Gew.-%, vorzugsweise mindestens 50 Gew.-% an Verbindungen der Formeln (Ia), (Ib) und (Ic) umfasst.

7. Erzeugnis gemäß Anspruch 6, **gekennzeichnet dadurch, dass** es ein Amidierungsprodukt oder ein trans-Amidierungsprodukt ist.

8. Erzeugnis gemäß einem der vorangehenden Ansprüche, **gekennzeichnet dadurch, dass** es eine Schmelztemperatur von weniger als oder gleich 35 °C, vorzugsweise weniger als oder gleich 25 °C, vorzugsweise weniger als oder gleich 10 °C, vorzugsweise weniger als oder gleich 0 °C aufweist.

9. Verfahren zur Herstellung eines Erzeugnisses gemäß einem der vorangehenden Ansprüche, umfassend einen Amidierungs- oder trans-Amidierungsschritt einer Verbindung der Formel R²R³NH und/oder HNR⁴R⁵ einer Mischung von mindestens zwei Verbindungen ausgewählt aus den Verbindungen der folgenden Formeln (I'a), (I'b) und (I'c):
R⁸OOC-A^{a}-COO R⁸ (I'a)
R⁸OOC-A^{b}-COO R⁸ (I'b)
R⁸OOC-A^{c}-COO R⁸ (I'c)
wobei R⁸ ein Wasserstoffatom oder ein C₁-C₆-Akyl ist, vorzugsweise ein Methyl.

10. Verwendung eines Erzeugnisses, wie in einem der Ansprüche 1 bis 8 definiert, als Lösemittel, Weichmacher oder Koaleszenzmittel.

11. Verwendung eines Erzeugnisses, wie in einem der Ansprüche 1 bis 8 definiert, in einer Pflanzenschutzmittelformulierung, in einer Reinigungsformulierung, in einer Beizformulierung, in einer Entfettungsformulierung, in einer Schmiermittelformulierung, in einer Beschichtungsformulierung, in einer Pigment- oder Tintenformulierung, oder in einer Kunststoffformulierung.

## Claims

1. A product comprising at least two diamide compounds selected from the diamide compounds of following formulae (Ia), (Ib), and (Ic):
R²R³NOC-A^{a}-CONR⁴R⁵ (Ia)
R²R³NOC-A^{b}-CONR⁴R⁵ (Ib)
R²R³NOC-A^{c}-CONR⁴R⁵ (Ic)
wherein:
- R², R³, R⁴, and R⁵, either identical or different, are groups selected from methyl, ethyl, propyl (n-propyl), isopropyl, n-butyl, isobutyl, n-pentyl, amyl, isoamyl, hexyl, cyclohexyl, hydroxyethyl, morpholine, piperazine or piperidine groups;
- A^{a} is the group of the following formula -CH₂-CH₂-(ethylene);
- A^{b} is the group of the following formula -CH₂-CH₂-CH₂- (n-propylene); and
- A^{c} is the group of the following formula -CH₂-CH₂-CH₂-CH₂-(n-butylene).

2. The product according to any of the preceding claims, **characterized in that** it comprises the three compounds of formula (Ia), (Ib) and (Ic).

3. The product according to any of the preceding claims, **characterized in that** it comprises, based on the total number of moles of diamides of formulae (Ia), (Ib), and (Ic):
- from 1 to 20%, preferably from 5 to 15%, by moles of diamide of formula (Ic)
- from 45 to 75%, preferably from 55 to 65%, by moles of diamide of formula (Ib),and
- from 15 to 45%, preferably from 20 to 33%, by moles of diamide of formula (Ia).

4. The product according to any of the preceding claims, **characterized in that**:
- the compound of formula (Ia) is the compound Me₂NOC-CH₂-CH₂-CONMe₂
- the compound of formula (Ib) is the compound Me₂NOC-CH₂-CH₂-CH₂-CONMe₂, and
- the compound of formula (Ic) is the compound Me₂NOC-CH₂-CH₂-CH₂-CH₂-CONMe₂.

5. The product of any of claims 1 to 3, **characterized in that**:
- the compound of formula (Ia) is the compound Et₂NOC-CH₂-CH₂-CONEt₂
- the compound of formula (Ib) is the compound Et₂NOC-CH₂-CH₂-CH₂-CONEt₂, and
- the compound for formula (Ic) is the compound Et₂NOC-CH₂-CH₂-CH₂-CH₂-CONEt₂.

6. The product according to any of the preceding claims, **characterized in that** it comprises at least 10% by weight, preferably at least 50% by weight, of compounds of formulae (Ia), (Ib) and (Ic).

7. The product according to claim 6, **characterized in that** it is an amidification or trans-amidification product.

8. The product according to any of the preceding claims, **characterized in that** it has a melting temperature of less than or equal to 35°C, preferably less than or equal to 25°C, preferably less than or equal to 10°C, preferably less than or equal to 0°C.

9. A method for preparing a product according to any of the preceding claims, comprising an amidification or trans-amidification step with a compound of formula R²R³NH and/or HNR⁴R⁵, of a mixture of at least two compounds selected from the compounds of the following formulae (I'a), (I'b) and (I'c):
R⁸OOC-A^{a}-COOR⁸ (I'a)
R⁸OOC-A^{b}-COOR⁸ (I'b)
R⁸OOC-A^{c}-COOR⁸ (I'c)
wherein R⁸ is a hydrogen atom or a C₁-C₆ alkyl preferably a methyl.

10. The use as a solvent, plasticizer or coalescence agent of a product as defined in any of claims 1 to 8.

11. The use of the product as defined in any of claims 1 to 8, in a phytosanitary formulation, in a cleaning formulation, in a stripping formulation, in a degreasing formulation, in a formulation of lubricants, in a coating formulation, in a formulation of pigments or ink or in a plastic formulation.
